# EUROPEAN PATENT SPECIFICATION

(11) **EP 3 258 999 B1**
(45) Date of publication and mention of the grant of the patent: **12.10.2022**
(21) Application number: 15790644.7
(22) Date of filing: 10.09.2015
(51) Int. Cl.: A61M 16/00, A61M 16/12

(54) **DEVICE FOR ADJUSTMENT AND/OR CONDITIONING OF THE CO2 CONTENT OF THE INHALED AIR**
VORRICHTUNG ZUR EINSTELLUNG UND/ODER KONDITIONIERUNG DES CO2-GEHALTS VON EINGEATMETER LUFT
DISPOSITIF POUR L'AJUSTEMENT ET/OU LE CONDITIONNEMENT DE LA TENEUR EN CO2 DE L'AIR INHALÉ

(30) Priority: 10.09.2014 HU P1400421
(43) Date of publication of application: 27.12.2017
(73) Proprietor: Pomoziné Gyarmathy, Ágnes, 2040 Budaörs (HU)
(72) Inventor: POMOZI, István, 2040 Budaörs (HU); SZÁZ, Dénes, 5000 Szolnok (HU)
(74) Representative: Giber, Janos
(86) International application number: PCT/HU2015/000064
(87) International publication number: WO 2016/038401

(56) References cited:
- EP-A1- 2 641 536
- WO-A1-98/41266
- CN-A- 102 488 952
- DE-A1- 2 543 266
- US-A- 5 320 093
- US-A- 5 957 129

## Description

The subject matter of the application is a device for adjustment and/or regulation of the CO₂, carbon dioxide content of the air inhaled by normal respiration for treatment hyperventilation comprising a by-pass element, leading the inhaled and exhaled air in two directions (10),for treatment of hyperventilation by increasing the CO₂ content of the inhaled air to the appropriate level to avoid hyperventilation by measuring the CO₂ level concentration for the same time but separately in the exhaled and inhaled air, using the CO₂ content of the air either directly exhaled or stored in an exhaled air vessel and/or using the CO₂ content of a CO₂ vessel.

The O₂, oxygen content of the inhaled and exhaled air can be measured at the same time also by using the device according to the invention. The O₂ content is not regulated directly, only by blending the fresh air or compressed air or O2 to the inhaled air using the data of the measurements.

In the course of a normal respiration the inhaled fresh air contains 78 % nitrogen, 21% oxygen and 1 % other gases, where the rate of CO₂ is evanescent (in case of clean air only 0,04 %). After the metabolic processes of respiration have passed off, the exhaled air contains only app. 16-17 % oxygen, and the level of CO₂ increases to 4-5 %.

During inspiration the oxygen is obtained from our atmosphere and during expiration the CO₂, generated in the course of the metabolism will be exhaled.

The oxygen then is bound by the haemoglobin of red-blood cell and this way it enters the blood circulation and is transported by the blood to the cells.

CO₂ is unloaded by the respiring cells in form of carbonic acid into the blood plasma. CO₂ then will be transported by the blood plasma tc the tiny air chambers where it will be secreted.

Lungs have therefore an important role in keeping the acid - base balance of the body.

However the carbonic acid content of the blood dissociate only partly, the task of the remaining part is to stimulate the respiratory centre. Respiration is regulated therefore by the chemical composition of the blood.

Respiration is regulated automatically (independently from our will) and dependently from our will. The centre of the automatic regulation is in the pons (part of the brainstem) and the upper Medulla, which is responsible for rhythmic respiration stimulated by the CO₂ (and not the lack of oxygen). Research and application of medical effects of CO₂ started already from the year of 1970. At the beginning, the medical effects of CO₂ were used for the treatment of different respiratory or asthmatic diseases, substituting the fluorocarbon based propellant gases, responsible for ingesting the active ingredient of medicine dispenser into the human organism (US 4137914). Later it has been found out, that the effectiveness of the medicine could be increased by giving CO₂ to the patient, moreover the use of CO₂ itself already causes the remission of the symptoms.

Several devices and procedures protected by patent came into general use, where by administering small dosages of CO₂ for longer time or bigger dosages of CO₂ or for shorter time symptoms of different diseases (headache, allergic cold, asthma, epilepsy, neuro diseases etc.) could be decreased temporarily (US 8646711, US 8398580, US 7836883, US 7748379, US 8959708). These kind of devices however can be used only for temporary treatment and only for symptoms. Handling some of them is difficult and also because of their size, simple, portable realization is not possible.

Although in the specifications of their patents, treatments of several different diseases were mentioned, there are no solutions, specially for treatment of hyperventilation syndrome of panic disorder patients, and their actuation is partly or fully non automated. In course of panic and asthmatic diseases hyperventilation (sudden rapid shallow breathing) is a frequent symptom, characterised by bronchospasms (spasms of the smooth muscles of the bronchi) and a strained state of diaphragm and other respiration essential and auxiliary muscles. Panic attack can be attended by psychic symptoms, like anxiety or unpleasant discomfort remitting to the unfavourable stimulated level of the cortex.

"It is well known fact from the physiology if too much air and oxygen gets into the human organism also too much carbon dioxide will be loosed. As the carbon dioxide and carbonic acid are the most important regulators of the base-acid balance (pH) this balance will be also upset.

First the respiratory alkalosis occurs, then, as a result of compensating processes due to the secondary carbic acid loss, a metabolic acidosis will be generated.

The respiratory alkalosis is accompanied and followed by the body tissue hypoxia. This is the result of the so called Bohr effect (Bohr shift), which means, that as soon as the pH becomes more alkaline due to a decrease in the CO₂ level, the oxygen will binds stronger to the blood, its release, will become more difficult, and it will become less accessible to human tissues." (http://www.natursziget.com/eletmod/20C71025butejkolegzes)

A drop in pH (more acidic) lowers the attraction of haemoglobin to oxygen. Because carbon dioxide reacts with water to produce carbonic acid, an active tissue will lower the pH of its surroundings and encourage haemoglobin tc give up extra oxygen, to be used in cellular.

Fellow workers of Southern Methodist University (Meuret et al, 2010) developed a new kind of respiratory therapy which reduces the feeling of panic and anxiety, known as CART (Capnometry-Assisted-Respiratcry Training). This new therapy teaches the patient the way of breathing to turn back the hyperventilation, characterised by the low level of carbon dioxide concentration in blood. Because of this fact, in spite of the popular belief, a deep breath can increase hyperventilation, and worsen the symptoms and state of patient. Patients suffering from advanced asthma are treated often by inhaling oxygen-rich air which could be markedly injurious during a fit.

The task of the invention should be a device using the results of CART respiratory technic, but using the subject device much less care and cooperation is needed from the patient.

Further task of the invention and application is a device, which is able to adjust and/or regulation the concentration of carbon dioxide of inhaled air automatically, so the state of hyperventilation cannot develop, the already developed hyperventilation stops and normal respiratory gets restored. As further task of the invention the device should be easily portable and usable for self-treatment by an average patient portable by the user's own strength in every-day life, outside of clinics and hospitals without any physical or medicinal help from outside.

Developing the invention we came to the conclusion, the device should comprise following elements:
- a by-pass element, leading the inhaled and exhaled air in two directions,
- an exhaled air pipe and an inhaled air pipe connected tc the by-pass element, where valves enabling one direction flow are placed and arranged in the pipes,
- a blending vessel connected to the inhaled air pipe comprising a fresh air input aperture and a CO₂ input aperture.

Such arrangement is cognisable from the specification of patent No. EP 1,267,978 where a CO₂ vessel is used but only for direct regulation of the inhaled air. The subject matter of the invention is completely different, because the device according to the cited patent is not a standalone, portable device it can be used only as controlling system for anaesthetic procedure as part of the anaesthetic device where a CO₂ absorber is used also.

There were some anaesthesiological devices for restoring respiratory to an anesthetized patient disclosed in the prior art but these solutions are not for stoppage and prevention of hyperventilation at any time or place when the fit of hyperventilation starts specially outside of hospitals and clinics (US 5320093, EP 2,641,536, WO 98/41266, DE 2543266 A1).

All these solutions are not regulating the CO₂ content of the inhaled air by measuring the CO₂ and O2 content of the inhaled and exhaled air separately, and using the exhaled air, CO2 gas and compressed air or O2 for the regulation by using blending vessel, CO2 vessel and vessel for compressed air or O2.

Patent US5320093 describes an anaesthesiological device for restoring the normal breathing of the patient after surgeries and anesthesia. The device regulates breathing by reducing exhaled CO2 content by using a CO2 absorber and the anaesthetic machine mixes the breathing gases (CO2, O2, N2O) in a blending area. The concentration of CO2 is measured only in the exhaled gas.

Patent US 5957129 describes another anaesthesiological device for regulating the patient's breathing and the delivery of anaesthetic medicines by evaporating the medicines in liquid form and mixing them into the breathing gas. The device reduces the CO2 content of the exhaled air by using a CO2 absorbent. For breathing gas regulation, a blending vessel is used where, in contrast to our solution, N2O, O2 and CO2 gases are mixed and then the evaporated medicines are added to the breathing gas mixture. The measurement of respiratory gas concentrations are not separated to exhaled and inhaled air sections. In our solution, we separated the measurement of inhaled and exhaled CO2 content for more detailed monitoring of the breathing and to set the inhaled CO2 content more accurately.

Both patents US 5320C93 and US 5957129 use a ventilator system to maintain constant breathing of the patient incapable of self-contained breathing, of normal respiration. These devices are designed for using only in hospitals with the supervision cf medical personnel. Based on this purpose, the devices are not portable by the patient and cannot be operated by the patient alone. The main purpose of these solutions is breathing support during and after anesthesia and not the stoppage and prevention of hyperventilation at any time or place when the fit of hyperventilation starts specially outside of hospitals and clinics.

The most general sense the subject matter and task of the invention is a device for adjustment and/or regulating the CO₂ content of the inhaled air where
- a CO₂ vessel is connected to the CO₂ input aperture,
- a measuring tool determining the CO₂ content of the exhaled air is connected to the exhaled air pipe,
- the output aperture of the measuring tool is connected to the input aperture cf a control unit.
- In case we would like to control the CO₂ content of the air inhaled from the blending vessel, a measuring tool, determining the CO₂ content of the inhaled air should be connected to the inhaled air pipe also.
- The output aperture of the measuring tool should be also connected to the control unit's input aperture.
- For further safety it could be advantageous if a measuring tool determining the O₂, oxygen content is connected to the inhaled air pipe and the exhaled air pipe also.
- An advantageous version of the device described in the application as subject matter of the invention is a device where the exhaled air pipe is connected to an exhaled air vessel, where one output aperture of the vessel is connected to the blending vessel the other output aperture is connected to a pipe opened to the atmosphere.

In this opened air pipe could be advantageously an opening - closing valve placed where the control input of the valve should be connected to the control unit.

A further advantageous version of the subject matter of the invention is, if the CO₂ vessel and the exhaled air vessel are connected through a shuttle valve to the blending vessel, where the control input cf the valve is connected to the output of the control unit.

For further advantageous solution to the output aperture of blending vessel a filter, advantageously a filter comprising more elements should be connected.

According to another advantageous version of the invention the exhaled air pipe is connected directly to the air pipe opened to the atmosphere.

In this case a CO₂ vessel and a compressed air or 02 vessel is connected to the blending vessel, so that these vessels are connected to a blending valve, arranged in the blending vessel, so an exhaled air vessel is not needed.

The device based on the invention comprises a by-pass element for leading the air, and to this by-pass element advantageously a breather pipe or breather mask could be connected directly or advantageously through a breathing-counter.

Advantageously the output of the breathing-counter is connected to the appropriate input of the control unit. The device based on the invention is fully automated CO₂ dispenser which keeps the concentration of CO₂ on the proper and-controlled level, not to develop hyperventilation state. In case of developed hyperventilation fit, the plus CO₂ dosage causes automatic breath reflex, which normalizes the respiratory of the patient again, ending the abnormal state.

The device based on the invention is monitoring continuously the concentration of CO₂ of the air exhaled by the patient, so reaching the normal level (there is no hyperventilation anymore) decreases or stops administering the CO₂-rich air automatically.

Solving the task of the invention mentioned before, the subject matter of invention uses the results of CART respiratory technic, but using the subject device much less care and cooperation is needed from the patient.

The subject matter of the invention will be described using the enclosed numbered figures with application examples in details, where
Figure 1 is the first version of the device based on the invention,
Figure 2 is the principled scheme of the second version of the device based on the invention
Figure 3-4 with the diagrams representing the measured and adjusted levels of CO₂

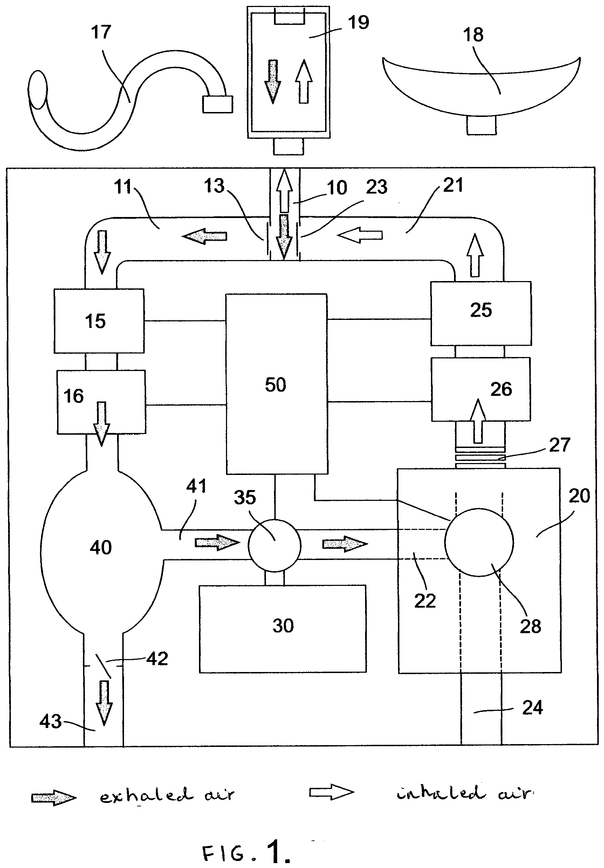

Some part of the device based on the invention represented on Figures 1-2 comprises the same elements and same structured elements. First these common elements will be described.

It is represented on both Figures thus, that the device comprises a by-pass element 10 leading the inhaled and exhaled air in two directions, an exhaled air pipe 11 and inhaled air pipe 21 both connected to the by-pass element 10. In both air pipes 11, 21 valves 13, 23 enabling one direction flow are placed and arranged.

To the inhaled air pipe 21 blending vessel 20 is connected comprising input aperture for fresh air 24 and input aperture for CO₂ 22. CO₂ vessel 30 is connected to the input aperture for CO₂ 22.

Measuring tool 15 determining the CO₂ content of the exhaled air is connected tc the exhaled air pipe 11.

The output aperture of the measuring tool 15 is connected to the input aperture of a control unit 50.

The device based on the invention comprises the by-pass element 10, and to this by-pass element 10 advantageously a breather pipe 17 or breather mask 18 could be connected directly or advantageously through a breathing counter 19.

Using the device occasionally the use of breath mask 18 could be appropriate, using continuously, the use of air pipe 17 is more advantageous. The end of air pipe 17 can be put into the blowhole of the treated patient and could be fixed if needed.

In both cases there is a possibility to use breath-counter 19, which allows monitoring and continuous measuring the respiratory rate.

Using the device based on the invention, CO₂ content of the exhaled air can be measured with the measuring tool 15, e.g., capnograph continuously and based on the results of this measurement the CO₂ content of the inhaled air can be adjusted and/or regulated automatically. Capable and portable capnograph for measuring the CO₂ concentration of the exhaled air is commercially easily available, e.g., under the name EMMA Capnograph.

Using a breathing-counter 19, there is a possibility for continuous monitoring and measuring respiratory rate. Therefore the output of the breathing-counter 19 should be connected to the appropriate input of the control unit 50. In this case the sign of changed respiratory rate could be used as trigger to start administering CO₂.

Changed respiratory rate is exhibitive of hyperventilation or diseased state. This possibility can be used primarily in case of patients suffering in heavy panic or asthmatic diseases, where hyperventilation fits appear often and unpredictably.

The CO2 content of the inhaled air can be adjusted and /or regulated more preciously if a measuring tool 25 is connected to the inhaled air pipe 21. The measuring tool 25 determines the CO₂ content of the inhaled air, and the output of this tool 25 is connected to the input of control unit 50.

According to the measurement results the control unit can regulate more preciously.

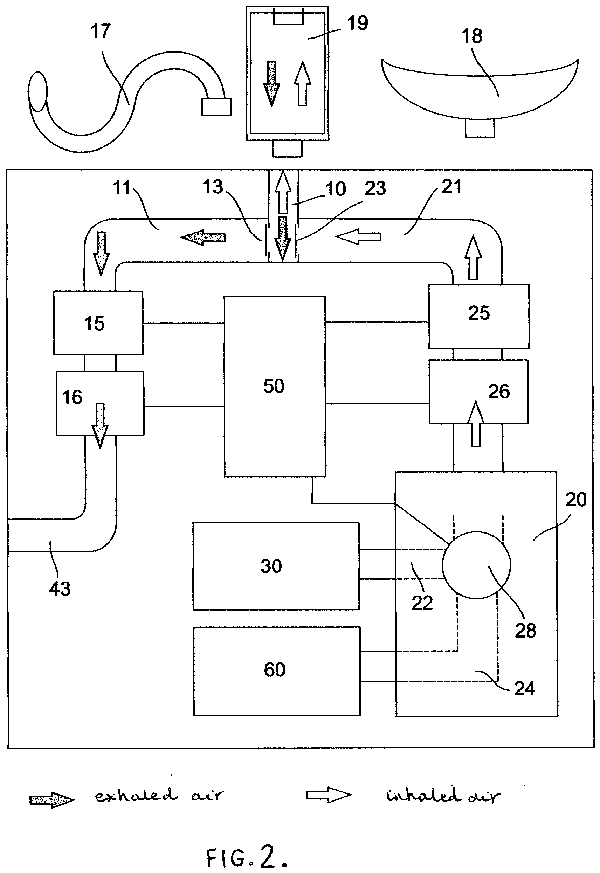

In the case of the application examples represented on Figures 1 and 2, O₂ content measuring tools 16 and 26 are connected to the exhaled air pipe 11 and the inhaled air pipe 21.

Based on the results of the measuring tools 16 and 26, the control unit 50 is able to adjust also the O₂ content of the inhaled air to a certain level, or to keep it on a certain level. To increase the O₂ content of the inhaled air, it is not sufficient to redirect the exhaled air, instead fresh air needs to be inducted from the atmosphere.

In the case of the application example shown on Figure 1, exhaled air pipe 11 is connected to the exhaled air vessel 40 that has two output apertures, one of them 41 is connected to the blending vessel 20, the other one 42 is connected to the air pipe 43 open to the atmosphere.

In the air pipe opened to the atmosphere 43 there is an opening - closing valve 42 placed where the control input is connected to control unit 50.

The CO₂ vessel 30 and the exhaled air vessel 40 are connected through a shuttle valve 35 to the blending vessel 20, where the control input of the valve 35 is connected to the output of the control unit 50.

Valve 42 and shuttle valve 35 gets a synchronised control with each other.

According to this control the valve 42 is closed when the shuttle valve 35 connects the exhaled air vessel 40 with the blending vessel 20, so the exhaled air cannot leave through the air pipe 43 opened to the atmosphere, but flows to the blending vessel 20.

In this case the fresh air in the inhaled air could be enriched with the CO₂ content of the exhaled air.

In such cases the content of CO₂ of the inhaled air could be maximum 4-5%.

When the shuttle valve 35 connects the CO₂ vessel 30 with the blending vessel 20, the valve 42 is in opened state, so the exhaled air is leaving through the air pipe 43 opened to the atmosphere and CO₂ flows from the CO₂ vessel 30 to the blending vessel 20.

In this case the fresh air in the inhaled air can be mixed with the CO₂ flowing out from the CO₂ vessel 30, so the CO₂ content of the inhaled air can be adjusted optionally. Another advantageous version of the invention could be where the shuttle valve 35 is substituted with a blending valve. In this case the blending valve can change the rate of the exhaled air and the CO₂ flowed from the CO₂ vessel 30 optionally, so the CO₂ content of the exhaled air could be increased and meanwhile the rate of other gases remain. In this case valve 42 should be controlled on a way that the unused exhaled air should leave to the atmosphere.

In the case of the application example shown on Figure 1 output aperture of blending vessel 20 is connected to filter 27 which filters the air getting into the inhaled air pipe 21. As the device makes possible for the patient and the air from atmosphere a direct contact, a system comprising three filters could secure, that the patient should not contact directly with any contamination. One filter for bacterium to filter the bacteria getting in with the air of atmosphere, one filter pollen to get rid of the pollens which could cause even an allergic fit, and a filter of dusk to filter the dusk and found in the air which could be often found in the city air.

In the case of application example of the device based on the invention shown on Figure 2 instead of exhaled air vessel 40 a compressed air or O2 vessel 60 is used, where exhaled air pipe 11 is connected directly to the air pipe 43 opened to the atmosphere.

In case of this version of the device the CO₂ vessel 30 and compressed air or O2 vessel 60 are connected to a blending valve 28 placed in the blending vessel 20.

The controllable input of the blending valve 28 is connected to the appropriate output of the control unit 50 also. The filtering of the inhaled air is not needed in this case, as the blending vessel 20 is not connected with the atmosphere and so the inhalable fresh air is not from there.

In case an O₂ vessel or compressed air vessel is used besides a CO₂ vessel, on the one hand it is possible to set the oxygen and carbon dioxide rate of the air inhaled by the patient more precisely within certain limits, on the other hand the patient can be completely isolated from the air of the air of the atmosphere, excluding the risk of bacterial and/or pollen pollution. According to this implementation, the patient does by no means re-inhale the air exhaled by them, instead the patient receives a completely fresh and sterile air produced in a regulated way, in which the rate of O₂ and CO₂ is constant and can be kept at a controlled level.

Measuring the O₂ concentration can be reasonable for several reasons. On the one hand, it provides additional information on the air exhaled by the patient, on the other hand monitoring the O₂ concentration also reduces the risk of asphyxia. Faint can be avoided with the help of an oxygen measuring tool, for example in case the O₂ level of the exhaled air is abnormally low (below 16 %), the inhaled air can be adjusted during the blending. This has to reach the O₂ level of the air inhaled in a normal situation, thus setting O₂ concentration of 20-22 % is optimal, to which CO₂ concentration, causing the elimination of hyperventilation, can also be added. In the case of the second implementation option, this can easily be adjusted with the help of the CO₂ and compressed air or O₂ vessels. However, in a closed system there is a close link between these two concentration values. By increasing the CO₂ concentration, the percentage of O₂ decreases, which might be dangerous- This can be avoided, if the gas composition in the compressed air container is not identical to the atmospheric air, but it has a higher O₂ rate, approximately 20-25%. In this case, even dosing 4-8 % CO₂, used during longer-term (few minutes) therapies, cannot reduce the O₂ concentration below 20 %. In the case of a short-term high CO₂ concentration therapy, this problem doesn't exist, since in this case the patient takes only a few breaths before he/she breaths fresh air again. In the case of the first implementation, it is important that in case exhaled oxygen level is low, the air is not recommended to redirect. In this case the fresh air of the atmosphere should be used, which contains the normal inspiration oxygen concentration. This can be mixed with an appropriate amount of therapeutic CO₂. In this case, instead of the shuttle valve 35, a blending valve 28 should be applied. In this case, due to the open system, the inhaled CO₂ concentration can be adjusted in such a way that the O2 level can also be kept at an appropriate value.

The size of the blending vessel 20 has to be selected in such a way that the mixed air is sufficient even for multiple respiratory cycles. The exhausted 02 or compressed air and CO₂ vessels always need to be replaced therefore it is desirable to also display the filling level of the vessels.

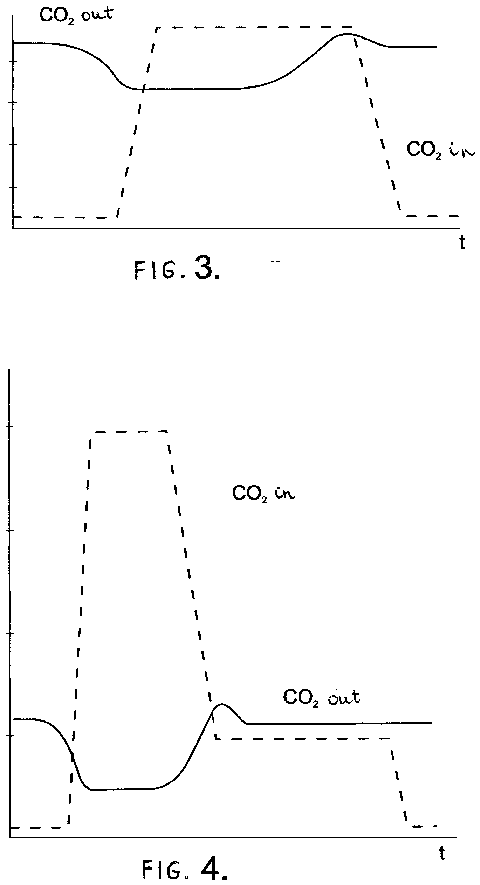

Figures 3 and 4 show the time diagrams of the CO₂ concentration figures measured in the inhaled and exhaled air in different therapy cycles using the device based on invention. In case of hyperventilation, the alveolar CO₂ concentration decreases which results in rapid breathing and increased respiratory rate. During the rapid breathing, ventilation becomes inappropriate, the body does not get sufficient amount of oxygen, and the CO₂ level decreases further. In this case, if the CO₂ concentration measured in the exhaled air gets below 4 % that can be considered as abnormal, and normally results in hyperventilation.

In the case represented on Figure 3 the CO₂ content of the exhaled air decreased only in a small compass, which means from starting value 4-5 % to 3-4 %. This small decreasing however could be a sign for a starting hyperventilation, when treatment should be started already. In such cases the CO₂ concentration of the inhaled air should be adjusted between 4-8 %, so that practically the exhaled air of the patient can be redirected and the CO₂ content of the exhaled air reused. The exhaled air can be enriched with CO₂ from the CO₂ vessel. Based on the measurement of the exhaled O₂ content the used procedure and control of the device based on the invention could be modified, so that the exhaled air will be redirected till the O2 concentration of the exhaled air remains above 16%. In case the O2 level of the exhaled air decreases below 16%, the redirection of exhaled air should be stopped, and the fresh (from atmosphere or compressed) air should be used enriching the inhaled air with CO₂ in a proper rate.

Beside or instead of the compressed air vessel O₂ vessel could be used also for adjusting the required O₂ level if needed.

Generally the treatment takes only a few minutes, which is enough to the restitution of the hyperventilation and the normal respiratory parameters.

In the case represented on Figure 4 the CO₂ content of the exhaled air decreased significantly, which means from starting value 4-5 % to 2 %. In such cases for treatment and to reach fast result, a higher CO₂ concentration of the inhaled air should be used but for shorter time (2-3 breaths). This higher CO₂ concentration could be 10-15% also. Short time using could be efficient to stop hyperventilation. After according to the measuring values hyperventilation has stopped, use of lower CO₂ concentrations could be used again till the end of the treatment with using the procedure described concerning Figure 3.

The device based on the subject invention occasionally could be used also for treatment acute symptoms or even continuous monitoring and treatment if needed.

The subject matter of the invention could be used also for therapeutic tasks in helping respiratory.

Generating automatic respiratory reflex the method could teach the patient right breathing, regulating by will after words, decreasing the chance to start a hyperventilation fit again. Completing the CART respiratory therapy with the device based on the invention the hyperventilation fits of patients who are panic diseased could even more effectively decreased and besides the fits of asthmatic patients could be decreased also.

As most of the asthmatic and panic diseased patient can feel the start of a fit directly before developing, with the recommended device based on the invention the CO₂ poor state causing the fit could be avoided. With subject procedure all the medicines for treatment and preventing hyperventilation could be replaced, which means lower costs to the patient for long time and the decreases drug loading and risks of side effects of medicines.

Concerning all these points of view the subject matter of the invention can improve the quality of life of the patients, suffering in diseases as described above, on a significant way.

Although the subject matter of the invention was described only through two application examples in details, it doesn't mean to limit the protection and scope of subject patent application to these examples.

As obvious for an expert the characteristics of the subject matter of the invention can be used itself or in other different combinations.

## Claims

1. Device for automatic adjustment and/or regulation of the CO₂, carbon dioxide content of the air inhaled by normal respiration for treatment hyperventilation ***characterised by***
comprising a
- by-pass element, leading the inhaled and exhaled air in two directions (10),
- an exhaled air pipe (11) and inhaled air pipe (21) connected to the by-pass element (10), where valves (13, 23) enabling one direction flow are placed and arranged in the pipes,
- a blending vessel (20) connected to the inhaled air pipe (21) comprising a fresh air input aperture (24) and a CO₂ input aperture (22),
- a CO₂ vessel (30) is connected to the CO₂ input aperture (22),
- a measuring tool determining the CO₂ content of the inhaled air (25) is connected to the inhaled air pipe (21),
- a measuring tool determining the CO₂ content of the exhaled air (15) is connected to the exhaled air pipe (11),
- the output apertures of the measuring tools (25, 15, 26, 16) are connected to the input aperture of a control unit (50),
- the output aperture of the control unit (50) is connected to the valve (28) adjusting the blending rate of blending vessel (20) and so adjusting the CO₂ content of the inhaled air by increasing the CO₂ content of the inhaled air to the appropriate level to avoid hyperventilation by using the CO₂ content of the air either directly exhaled or stored in the exhaled air vessel (40) and/or using the CO₂ content of the CO₂ vessel (30), and/or using the O₂ content of the fresh air and/or of a compressed air or O2 vessel (60), and
- to the bypass element (10) of the device there is a breather pipe, or a breather mask connected directly or through a breathing-counter (19) and the output of the breathing-counter is connected to the appropriate input of the control unit (50).

2. Device according to claim 1 ***characterised by***
that a measuring tool, determining the CO₂ content of the inhaled air (25) is connected to the inhaled air pipe (21), and the output aperture of the measuring tool is connected to the input aperture of the control unit (50).

3. Device according to any of claims 1 or 2 ***characterised by*** that a measuring tool determining the oxygen content (16, 26) is connected to the exhaled air pipe (11) and to inhaled air pipe (21) also.

4. Device according to any of claims 1 to 3 ***characterised by** that* the exhaled air pipe is (11) connected to an exhaled air vessel (40), where one output aperture (41) of the vessel is connected to the blending vessel (20) the other output aperture (42) is connected to a pipe opened to the atmosphere (43).

5. Device according to any of the claims 1 to 4 ***characterised by***
that in air pipe opened to the atmosphere (43) is an opening - closing valve (42) placed where the control input of the valve should be connected to the control unit (50).

6. Device according to any of claims 1 to 5 ***characterised by*** that the CO₂ vessel (30) and the exhaled air vessel (40) are connected through a shuttle valve (35) to the blending vessel (20), where the control input of the valve (35) is connected to the output of the control unit (50).

7. Device according to any of claims 1 to 3 and 5 ***characterised by*** that the CO₂ vessel (30) and the exhaled air pipe (11) are connected through a shuttle valve (35) to the blending vessel (20), where the control input of the valve (35) is connected to the output of the control unit (50).

8. Device according to any of claims 1 to 7 ***characterised by*** that the output aperture of blending vessel (20) a filter (27) is connected.

9. Device according to claim 8 ***characterised by*** that the filter (27) comprises more elements.

10. Device according to any of claims 1 to 3 ***characterised by*** that the exhaled air pipe (11) is connected directly to the air pipe opened to atmosphere (43) .

11. Device according to claim 10 ***characterised by*** that a CO2 vessel (30) and a compressed air vessel (60) is connected to the blending vessel (20), so that these vessels are connected to a blending valve (28), placed in the blending vessel (20).

12. Device according to claim 11 ***characterised by*** that instead of the compressed air vessel (60) an O₂ vessel (60) is connected to the blending vessel (20).

13. Device according to any of claims 1 to 12 ***characterised by*** that to the by-pass element (10) or to a breathing counter (19) a breather pipe (17) is connected.

14. Device according to any of claims 1 to 12 ***characterised by*** that to the by-pass element (10) or to a breathing counter (19) a breather mask (18) is connected.

15. Device according to any of claims 1 to 14 ***characterised by*** that to the by-pass element (10) a breathing-counter (19) is connected, and the output of the breathing-counter is connected to the appropriate input of the control unit (50).

## Patentansprüche

1. Vorrichtung zur automatischen Einstellung und/oder Regulierung des CO₂-, Kohlendioxidgehalts der bei normaler Atmung eingeatmeten Luft zur Behandlung von Hyperventilation,
***dadurch gekennzeichnet, dass***
sie Folgendes umfasst
- ein Umgehungselement, das die eingeatmete und ausgeatmete Luft in zwei Richtungen (10) führt,
- eine Röhre für ausgeatmete Luft (11) und eine Röhre für eingeatmete Luft (21), die mit dem Umgehungselement (10) verbunden sind, wobei Ventile (13, 23), die einen Strom in eine Richtung ermöglichen, platziert und in den Röhren angeordnet sind,
- einen Mischbehälter (20), der mit der Röhre für eingeatmete Luft (21) verbunden ist, umfassend eine Frischluftzufuhröffnung (24) und eine CO₂-Zufuhröffnung (22),
- einen CO₂-Behälter (30), der mit der CO₂-Zufuhröffnung (22) verbunden ist,
- ein Messwerkzeug, das den CO₂-Gehalt der eingeatmeten Luft (25) bestimmt, das mit der Röhre für eingeatmete Luft (21) verbunden ist,
- ein Messwerkzeug, das den CO₂-Gehalt der ausgeatmeten Luft (15) bestimmt, das mit der Röhre für ausgeatmete Luft (11) verbunden ist,
- wobei die Ausgabeöffnungen der Messwerkzeuge (25, 15, 26, 16) mit der Zufuhröffnung einer Steuereinheit (50) verbunden sind,
- wobei die Ausgabeöffnung der Steuereinheit (50) mit dem Ventil (28) verbunden ist, das die Mischrate des Mischbehälters (20) einstellt und so den CO₂-Gehalt der eingeatmeten Luft durch Erhöhen des CO₂-Gehalts der eingeatmeten Luft auf das geeignete Niveau einstellt, um Hyperventilation zu vermeiden, indem der CO₂-Gehalt der Luft, die entweder direkt ausgeatmet wird oder im Behälter für ausgeatmete Luft (40) gespeichert wird, verwendet wird und/oder der CO₂-Gehalt des CO₂-Behälters (30) verwendet wird und/oder der O2-Gehalt der Frischluft und/oder einer Druckluft oder O₂-Behälters (60) verwendet wird, und
- mit dem Umgehungselement (10) der Vorrichtung eine Atmungsröhre oder eine Atmungsmaske direkt oder über einen Atmungszähler (19) verbunden ist und die Ausgabe des Atmungszählers mit der geeigneten Zufuhr der Steuereinheit (50) verbunden ist.

2. Vorrichtung nach Anspruch 1, ***dadurch gekennzeichnet,***
**dass** ein Messwerkzeug, das den CO₂-Gehalt der eingeatmeten Luft (25) bestimmt, mit der Röhre für eingeatmete Luft (21) verbunden ist und die Ausgabeöffnung des Messwerkzeugs mit der Zufuhröffnung der Steuereinheit (50) verbunden ist.

3. Vorrichtung nach einem der Ansprüche 1 oder 2, ***dadurch gekennzeichnet,* dass** ein Werkzeug, das den Sauerstoffgehalt (16, 26) bestimmt, mit der Röhre für ausgeatmete Luft (11) und ebenfalls mit der Röhre für eingeatmete Luft (21) verbunden ist.

4. Vorrichtung nach einem der Ansprüche 1 bis 3, ***dadurch gekennzeichnet,* dass** die Röhre für ausgeatmete Luft (11) mit einem Behälter für ausgeatmete Luft (40) verbunden ist, wobei eine Ausgabeöffnung (41) des Behälters mit dem Mischbehälter (20) verbunden ist, die andere Ausgabeöffnung (42) mit einer zur Atmosphäre geöffneten Röhre (43) verbunden ist.

5. Vorrichtung nach einem der Ansprüche 1 bis 4,
***dadurch gekennzeichnet,* dass** in der zur Atmosphäre geöffneten Röhre (43) ein Öffnungs-Schließventil (42) platziert ist, wobei die Steuerzufuhr des Ventils mit der Steuereinheit (50) verbunden werden sollte.

6. Vorrichtung nach einem der Ansprüche 1 bis 5, ***dadurch gekennzeichnet,* dass** der CO₂-Behälter (30) und der Behälter für ausgeatmete Luft (40) über ein Wechselventil (35) mit dem Mischbehälter (20) verbunden sind, wobei die Steuereinheit des Ventils (35) mit der Ausgabe der Steuereinheit (50) verbunden ist.

7. Vorrichtung nach einem der Ansprüche 1 bis 3 und 5, ***dadurch gekennzeichnet,* dass** der CO₂-Behälter (30) und die Röhre für ausgeatmete Luft (11) über ein Wechselventil (35) mit dem Mischbehälter (20) verbunden sind, wobei die Steuereinheit des Ventils (35) mit der Ausgabe der Steuereinheit (50) verbunden ist.

8. Vorrichtung nach einem der Ansprüche 1 bis 7, ***dadurch gekennzeichnet,* dass** die Ausgabeöffnung des Mischbehälters (20) ein Filter (27) verbunden ist.

9. Vorrichtung nach Anspruch 8, ***dadurch gekennzeichnet,* dass** der Filter (27) mehrere Elemente umfasst.

10. Vorrichtung nach einem der Ansprüche 1 bis 3, ***dadurch gekennzeichnet,* dass** die Röhre für ausgeatmete Luft (11) direkt mit der zur Atmosphäre geöffneten Röhre (43) verbunden ist.

11. Vorrichtung nach Anspruch 10, ***dadurch gekennzeichnet,* dass** ein CO₂-Behälter (30) und ein Druckluftbehälter (60) derart mit dem Mischbehälter (20) verbunden ist, dass diese Behälter mit einem Mischventil (28) verbunden sind, das im Mischbehälter (20) platziert ist.

12. Vorrichtung nach Anspruch 11, ***dadurch gekennzeichnet,* dass** ein O₂-Behälter (60) anstatt eines Druckluftbehälters (60) mit dem Mischbehälter (20) verbunden ist.

13. Vorrichtung nach einem der Ansprüche 1 bis 12, ***dadurch gekennzeichnet,* dass** mit dem Umgehungselement (10) oder mit einem Atmungszähler (19) eine Atmungsröhre (17) verbunden ist.

14. Vorrichtung nach einem der Ansprüche 1 bis 12, ***dadurch gekennzeichnet,* dass** mit dem Umgehungselement (10) oder mit einem Atmungszähler (19) eine Atmungsmaske (18) verbunden ist.

15. Vorrichtung nach einem der Ansprüche 1 bis 14, ***dadurch gekennzeichnet,* dass** mit dem Umgehungselement (10) ein Atmungszähler (19) verbunden ist und die Ausgabe des Atmungszählers mit der geeigneten Zufuhr der Steuereinheit (50) verbunden ist.

## Revendications

1. Dispositif pour l'ajustement et/ou la régulation automatique de la teneur en dioxyde de carbone, CO₂, de l'air inhalé par une respiration normale pour le traitement de l'hyperventilation
***caractérisé en ce qu'***
il comprend
- un élément de dérivation, conduisant l'air inhalé et exhalé dans deux directions (10),
- un tuyau d'air exhalé (11) et un tuyau d'air inhalé (21) raccordés à l'élément de dérivation (10), où des soupapes (13, 23) permettant un flux de direction sont placées et agencées dans les tuyaux,
- un récipient de mélange (20) raccordé au tuyau d'air inhalé (21) comprenant une ouverture d'entrée d'air frais (24) et une ouverture d'entrée de CO₂ (22),
- un récipient de CO₂ (30) est raccordé à l'ouverture d'entrée de CO₂ (22),
- un outil de mesure déterminant la teneur en CO₂ de l'air inhalé (25) est raccordé au tuyau d'air inhalé (21),
- un outil de mesure déterminant la teneur en CO₂ de l'air exhalé (15) est raccordé au tuyau d'air exhalé (11),
- les ouvertures de sortie des outils de mesure (25, 15, 26, 16) sont raccordées à l'ouverture d'entrée d'une unité de commande (50),
- l'ouverture de sortie de l'unité de commande (50) est raccordée à la soupape (28) ajustant le taux de mélange du récipient de mélange (20) et ajustant ainsi la teneur en CO₂ de l'air inhalé en augmentant la teneur en CO₂ de l'air inhalé jusqu'au niveau approprié pour éviter l'hyperventilation en utilisant la teneur en CO₂ de l'air soit directement exhalé soit stocké dans le récipient d'air exhalé (40) et/ou en utilisant la teneur en CO₂du récipient de CO₂ (30), et/ou en utilisant la teneur en 02 de l'air frais et/ou d'un air comprimé ou du récipient d'O₂ (60), et
- à l'élément de dérivation (10) du dispositif, il y a un tuyau de respirateur ou un masque de respirateur raccordé directement ou par l'intermédiaire d'un compteur de respiration (19) et la sortie du compteur de respiration est raccordée à l'entrée appropriée de l'unité de commande (50).

2. Dispositif selon la revendication 1, ***caractérisé en ce qu'***
un outil de mesure, déterminant la teneur en CO₂ de l'air inhalé (25), est raccordé au tuyau d'air inhalé (21), et l'ouverture de sortie de l'outil de mesure est raccordée à l'ouverture d'entrée de l'unité de commande (50).

3. Dispositif selon l'une quelconque des revendications 1 ou 2, **caractérisé en ce qu'**un outil de mesure déterminant la teneur en oxygène (16, 26) est raccordé au tuyau d'air exhalé (11) et également au tuyau d'air inhalé (21).

4. Dispositif selon l'une quelconque des revendications 1 à 3, **caractérisé en ce que** le tuyau d'air exhalé (11) est raccordé à un récipient d'air exhalé (40), où une ouverture de sortie (41) du récipient est raccordée au récipient de mélange (20), l'autre ouverture de sortie (42) est raccordée à un tuyau ouvert sur l'atmosphère (43).

5. Dispositif selon l'une quelconque des revendications 1 à 4,
***caractérisé en ce que**,* dans le tuyau d'air ouvert sur l'atmosphère (43), il y a une soupape ouverture-fermeture (42) placée où l'entrée de commande de la soupape devrait être raccordée à l'unité de commande (50).

6. Dispositif selon l'une quelconque des revendications 1 à 5, ***caractérisé en ce que*** le récipient de CO₂ (30) et le récipient d'air exhalé (40) sont raccordés par l'intermédiaire d'une soupape-navette (35) au récipient de mélange (20), où l'entrée de commande de la soupape (35) est raccordée à la sortie de l'unité de commande (50).

7. Dispositif selon l'une quelconque des revendications 1 à 3 et 5, ***caractérisé en ce que*** le récipient de CO₂ (30) et le tuyau d'air exhalé (11) sont raccordés par l'intermédiaire d'une soupape-navette (35) au récipient de mélange (20), où l'entrée de commande de la soupape (35) est raccordée à la sortie de l'unité de commande (50).

8. Dispositif selon l'une quelconque des revendications 1 à 7, ***caractérisé en ce que**,* à l'ouverture de sortie du récipient de mélange (20), un filtre (27) est raccordé.

9. Dispositif selon la revendication 8, ***caractérisé en ce que*** le filtre (27) comprend plus d'éléments.

10. Dispositif selon l'une quelconque des revendications 1 à 3, **caractérisé en ce que** le tuyau d'air exhalé (11) est raccordé directement au tuyau d'air ouvert sur l'atmosphère (43).

11. Dispositif selon la revendication 10, *caractérisé en ce qu*'un récipient de CO₂ (30) et un récipient d'air comprimé (60) sont raccordés au récipient de mélange (20), de sorte que ces récipients sont raccordés à une soupape de mélange (28), placée dans le récipient de mélange (20).

12. Dispositif selon la revendication 11, ***caractérisé en ce que**,* à la place du récipient d'air comprimé (60), un récipient d'O₂ (60) est raccordé au récipient de mélange (20).

13. Dispositif selon l'une quelconque des revendications 1 à 12, ***caractérisé en ce que**,* à l'élément de dérivation (10) ou à un compteur de respiration (19), un tuyau de respirateur (17) est raccordé.

14. Dispositif selon l'une quelconque des revendications 1 à 12, ***caractérisé en ce que**,* à l'élément de dérivation (10) ou à un compteur de respiration (19), un masque de respirateur (18) est raccordé.

15. Dispositif selon l'une quelconque des revendications 1 à 14, ***caractérisé en ce que**,* à l'élément de dérivation (10), un compteur de respiration (19) est raccordé et la sortie du compteur de respiration est raccordée à l'entrée appropriée de l'unité de commande (50).
